# EUROPEAN PATENT SPECIFICATION

(11) **EP 1 075 186 B1**
(45) Date of publication and mention of the grant of the patent: **24.11.2010**
(21) Application number: 99921794.6
(22) Date of filing: 06.05.1999
(51) Int. Cl.: A61K 31/555, A61P 31/22

(54) **METHOD OF HSV PROPHYLAXIS**
VERFAHREN ZUR HSV-PROPHYLAXE
METHODE DE TRAITEMENT PROPHYLACTIQUE DE L'INFECTION A HSV

(30) Priority: 06.05.1998 US 84448 P
(43) Date of publication of application: 14.02.2001
(73) Proprietor: The Children's Hospital Research Foundation, Cincinnati, OH 45229 (US)
(72) Inventor: BOURNE, Nigel-Children's Hospital Medical Center, Cincinnati, OH 45229 (US); STANBERRY, Lawrence, R., Cincinnati, OH 45229 (US)
(74) Representative: Wainwright, Jane Helen
(86) International application number: PCT/US1999/010091
(87) International publication number: WO 1999/056552

(56) References cited:
- WO-A-93/11140
- US-A- 5 049 557
- US-A- 5 142 076
- US-A- 5 756 491
- SCHWARTZ J.A. ET AL JOURNAL OF VIROLOGY vol. 75, no. 9, May 2001, pages 4117 - 4128
- BROCK ET AL: 'Biology of Microorganisms', 1991, PRENTICE HALL, SIXTH ED. * the whole document *
- FIELDS ET AL: 'VIROLOGY, StartDateMarker 2002, EndDateMarker Fourth Edition, Chapter 15', vol. 1, 2002 pages 394-395 - 404-405 * the whole document *
- DOMS ET AL: 'Viral Entry Denied' NEW ENGLAND JOURNAL OF MEDICINE vol. 351, 2004, pages 743 - 744
- SMITH ET AL: 'Virology: On Virus entry into animal cells' SCIENCE vol. 304, 2003, page 237

## Description

### BACKGROUND OF THE INVENTION

The present invention relates to and the use of metallo-organic cobalt compounds in the prophylactic treatment of subjects to prevent herpes virus infections.

It has been discovered that certain conditions and diseases, e.g., inflammation, burns, wounds, and diseases caused by bacteria and fungi in mammalian species can be treated with certain complexes of cobalt having the structure: 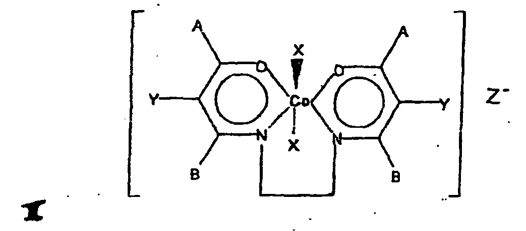 wherein each A may be the same or different and is an alkyl group, a phenyl group or a substituted derivative of a phenyl group;
wherein each Y may be the same or different and is hydrogen, an unbranched alkyl group, a halide or a group having the structure 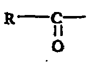 wherein R is hydrogen, an alkoxide group, and alkyl group, or OH;
wherein each B may be the same or different and each is hydrogen or an alkyl group; wherein each X may be the same or different and each is a water soluble group having weak to intermediate ligand filed strength; and
Z⁻ is a soluble, pharmaceutically acceptable negative ion.

U. S. Patent 5,142,076, discloses the use of the foregoing described compounds as treatment for viral diseases.

WO 93/11140 relates to the use of compositions comprising organometallic cobalt compounds for the treatment of subjects for conditions and diseases caused by viruses and viral infections.

Post-published evidence (Schwarz et al., 2001, J. Virology, 75: 4117-4128) demonstrate that compounds of the class claimed in the present application exert their antiviral action by inhibiting entry of enveloped viruses into host cells, but have no effect on the accumulation of viral proteins after initiation of infection.
Doms et al discuss in a post-published article in N. Engl. J. Med., 2004, 351: 743-4, a new class of antiviral compounds that block entry of the virus into the cell.

Today, virus infections are known to be significant causes of morbidity and mortality in human and veterinary medicine. Many of these diseases are untreatable or the available therapies are not entirely satisfactory and only provide minimal clinical response. New prophylactic treatments would decrease the incidence of these diseases and improve overall health.

### SUMMARY OF THE INVENTION

I have discovered a prophylactic use for the compound having the structure: 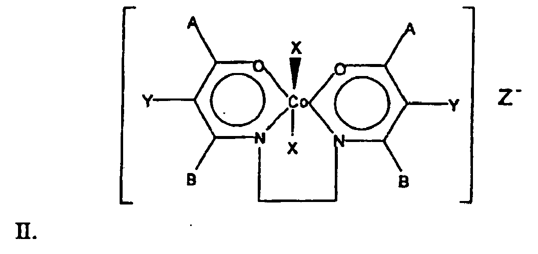 wherein wherein
- each A: is a methyl group;
- each Y: is hydrogen;
- each B: is a methyl group;
- Z⁻: is a bromine ion; and
- each X: is a moiety having the formula: 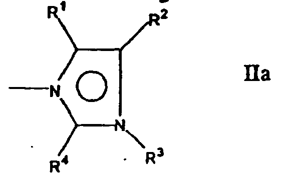
wherein

each of R¹, R² and R³ is hydrogen and R⁴ is a methyl group.

The compound having the structure of Formula II exhibit prophylactic efficacy when applied as a topical composition to the contact site prior to contact with herpes virus, and/or by inactivating herpes virus exposed to the composition.

### BRIEF DESCRIPTION OF THE DRAWING

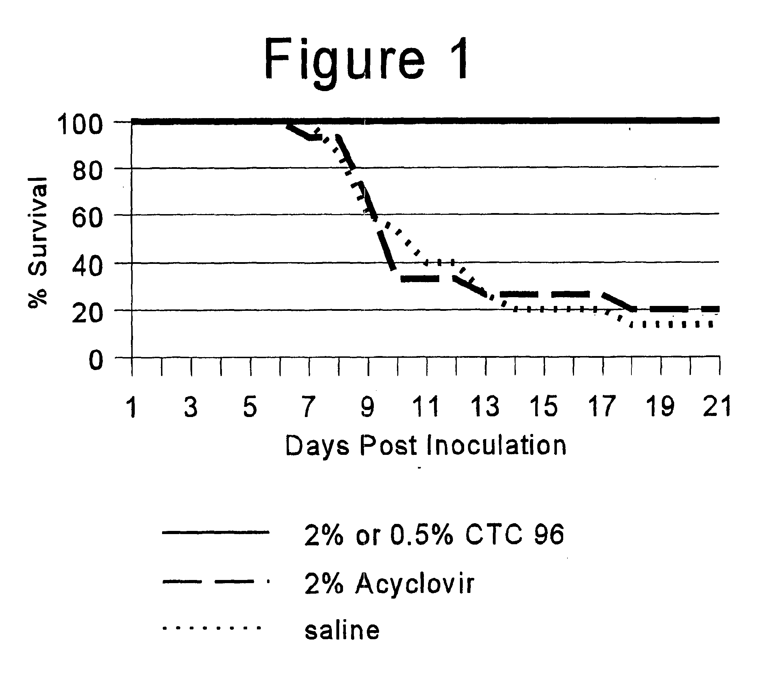

Figure 1 is a graph depicting a study of the prophylactic properties of topical application of compound having the structure of Formula II and comparison compounds.

### DETAILED DESCRIPTION OF THE INVENTION

The compound used may be crystallized with numerous counter-anions. Counter-anions which are pharmaceutically acceptable and are water soluble, such as, halide ions, PF₆⁻ and BF₄⁻, are preferred. The bromide and chloride salts of the present compounds are the most preferred because they are more water soluble than other salts of the compounds.

As discussed above, A is a methyl group.

Y is hydrogen.

B is a methyl.

X is an imidazole group linked to the cobalt atom through a nitrogen of the ring. The imidazole has appendant groups R¹, R², R³ and R⁴ wherein each of R¹, R² and R³ is hydrogen and R⁴ is a methyl group.

The following Table provides the structures of compounds related to the present invention. Compound 23, disclosed in the U. S. Patent 5,142,076 as exhibiting antiviral activity, is included as a comparison in the examples that follow. Compound 96 is the compound of the present invention.

In the following diagram, B is, in each case, methyl, and A, Y, X and Z⁻ refer to those symbols as used in structure II.

| **COMPOUND** | **Y** | **X** | **Z** | **A** |
|---|---|---|---|---|
| 23 | H | -NH₃ | Cl | -CH₃ |
| 76 | H | 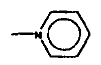 | Br | -CH₃ |
| 82 | H | 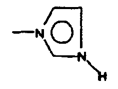 | Cl | CH₃ |
| 93 | Cl | 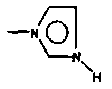 | Br | -CH₃ |
| 96 | H | 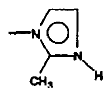 | Br | -CH₃ |
| 97 | H | 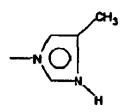 | Br | -CH₃ |
| 98 | H | 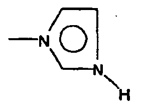 | Br | C₆H₅ |
| 100 | Cl | 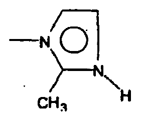 | Br | -CH₃ |
| 101 | Cl | 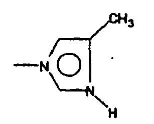 | Br | -CH₃ |
| 102 | H | 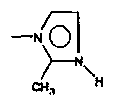 | Cl | C₆H₅ |
| 109 | H | 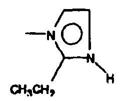 | Cl | -CH₃ |

The compositions used comprise a pharmaceutically acceptable carrier and the compound as defined above in a herpes virus prophylactic effective amount. As used herein, the expressions herpes virus prophylactic effective amount, dosage or regimen means that amount, dosage or regimen which results in a sufficient concentration of the particular compound at an appropriate site to prevent herpes virus disease. By appropriate site, it is meant a site which potentially contains herpes virus or is an area of a subject of potential exposure to herpes virus disease or is an area of a subject that has been exposed to herpes virus disease but as a result of such exposure, the subject has not yet acquired herpes virus disease. As used herein, the expression acquired herpes virus disease means that the subject, in fact, has the disease and can no longer be treated prophylactically to prevent the disease and must be treated therapeutically to ameliorate the disease.

The compound and compositions may be used in preventing infections caused by a variety of herpes viruses. The present invention includes the inventive compositions wherein the composition contains the compound as defined hereinabove in a prophylactic amount which is effective against the specific herpes virus. Known viruses of clinical significance are disclosed in PDR Medical Dictionary, 1st Edition, Williams & Wilkins, pp. 1939-1947, (1995); Virology, B.N. Fields, D.M. Knipe, P.M. Howley, R.M. Chanock, J.L. Melnick, T.P. Monath, B. Roizman and S.E. Straus, Lippincott-Raven Press, N.Y. (1996). See also Antiviral Agents and Viral Diseases of Man, George J. Galasso, Richard J. Whitley, and Thomas C. Merigan, Ed., 4° Edition, Lippincott-Raven Press, N.Y. (1997).

The compound is particularly effective against, *inter alia,* HSV-1, HSV-2, CMV, VZV, HHV-6, EBV, and the like.

For topical administration, the composition may be placed in a pharmaceutically acceptable saline solution, ointment, salve, cream or the like. The compound used in the present invention is water soluble, although the degree of solubility may vary and may be dissolved in a number of conventional pharmaceutically acceptable carriers. Suitable carriers include polar, protic solvents, such as, water, or normal saline. The compound may also be suspended in a suspension medium that is not miscible with water, for example, petrolatum.

When the compound of formula II is to be administered by the topical route for prevention of infection, i.e., prophylaxis or disinfection, the concentration in the saline, ointment, salve, creme, or the like can vary from about 0.00005 to about 5% by weight. A preferred concentration range lies between about 0.0005 and about 2% by weight. Typically, the topical composition shows prophylactic effect when applied to the contact site from about 1 hour before contact with the virus to about 6 hours after contact with the virus. Preferably, the topical composition is applied within five minutes of contact with the herpes virus. Particularly, the inventive compositions can be applied intravaginally for the prevention of sexually transmitted diseases. The topical composition containing the inventive compound could, for example, be coated on a condom or other sexual barrier device.

General methods for the synthesis of the compound are described in U.S. Patent No. 5,049,557, referred to hereinabove. As noted therein, the reaction of Co(II) complexes with molar oxygen has been studied extensively (see, R.S. Drago and B. R. Corden, Acc. Chem. Res., 1980, 13, 353 & E. C. Niederhoffer, J. H. Timmons and A.E. Martell, Chem. Rev. 1984, 84, 137). Normally, cobalt (II) forms 2:1 peroxo bridged complexes in aqueous solutions (see E. C. Niederhoffer, J.H. Timmons and A. E. Martell, Chem. Rev. 1984, 84, 137). In recent years, a number of Co(II) complexes have been reported to give 1:1 cobalt-oxygen adducts at room temperature. These complexes usually contain ligands which when bound to Co(II) give rise to a low spin planar geometry. Addition of base and O₂ to these complexes leads to the formation of octahedral complexes where the base and the O₂ occupy axial positions (see, A. Summerville, R.D. Jones, B.M. Hoffman and F. Basolo, J.Chem. Educ., 1979, 56, 3, 157).

On the basis of measurements utilizing a variety of physical techniques, it is now a well-accepted fact that the most accurate electronic structure description of the Co:O₂ moiety is a Co(III) ion bound to O₂⁻, where the actual amount of Co → O₂ electron transfer depends on the nature of the ligand and the donor set (see, A. summerville, R. D. Jones, B.M. Hoffman and F. Basolo, J. Chem. Educ. 1979, 56, 3 157, & D. Getz, E. Malmud, B. L. Silver and Z. Dori, J. Am Chem. Soc., 1975, 97, 3846). It has been shown that electron transfer increases with increase of the ligand field strength (see, R. S. Drago and B. R. Corden, Ace. Chem. Res., 1980, 13, 353). This can be easily understood from the molecular orbital diagram depicted in Fig. 1 of U. S. Patent 5,049,557 and the description therein.

The following examples illustrate the present invention. Compound 96 is the compound of the invention. The methods used in the examples are described in the following references:
For *in vivo* activity and toxicity of antiviral drugs for herpes viruses, see Antiviral Agents and Viral Diseases of Man, supra. In particular, Chapter 3, Preclinical Evaluation of Antiviral Agents; In vitro and Animal Model Testing by Dr. Earl R. Kern; and Chapter 6, Major Ocular Viral Infections by Dr. Deborah Paran-Langston.

### EXAMPLE 1

### In vitro Assays With HSV-1 Virus

*In vitro* asays were carried out to determine the direct virucidal efficacy of the compound to be tested, the potential toxicity of the compound and the intracellular anti-viral activity. The tests were carried out as follows:

### A. Viral Strain Used

HSV1 McKrae Strain was diluted to a final concentration of 10⁵ PFU/ml in minimal essential medium (MEM). Hereinafter, this dilution is referred to as the HSV-1 suspension for convenience.

### B. Preparation of Solutions of Inventive Compounds to be Tested

A stock solution of each compound to be tested at a concentration of 5 mg/ml was prepared. The stock solution was serially diluted in Hank's Balanced Salt Solution (HBSS) to obtain final drug concentrations of 5, 2, 1, 0.5, 0.1, 0.01, 0.001 and 0.0001 mg/ml. At the time of these experiments, certain of the compounds were observed to be insoluble at the higher concentrations. In such cases, the highest dissolved concentration that could be obtained was utilized. It was subsequently found that the insolubility was due solely to the technique used for solubilization, i.e., insufficient stirring was used. With sufficient stirring, all the compounds completely dissolved.

### C. Determination of Direct Virucidal Efficacy

Each of the drug solutions were mixed with the HSV-1 suspension in a 1:1 ratio. The drug and HSV-1 suspension mixture was incubated at 37°C for thirty minutes with agitation every then minutes. After the incubation, fifty microliter aliquots were overlaid onto triplicate confluent human foreskin fibroblast (HFF) cell monolayers. The inoculum was absorbed for thirty minutes and then a medium containing the appropriate drug concentration in MEM was added to the monolayers to a final volume of 0.5 mililiters per culture well. The monolayers were incubated at 37°C. The developing HSV-1 cytopathology was monitored daily for two days by inverted light microscopy. Titers were calculated by multiple regression analysis.

All data is presented as the average PFU/ml reduction at 24 and/or 48 hours after inoculation.

### D. Controls

Sham-incolulated, non-treated HFF cell monolayers were included along with the drug-treated monolayers as "cell monolayer controls".
1. HSV-1 inoculated, non-compound treated HFF monolayers were included as "positive controls"; and
2. Sham-inoculated compound treated HFF monlayers were included to demonstrate potential "toxicity effects" of the compounds.

### E. Determination of Intracellular Antiviral Activity

HFF cell monolayers were inoculated with the HSV-suspension prepared as above by absorption for thirty minutes at 37°C. After absorption of the virus, the inoculum was aspirated from the cell monolayers and the HSV-1 infected monolayers were rehydrated with medium containing the appropriate concentration of the solution fo the compound by adding 500 microliters of each compound concentration to triplicate HSV-1 infected monolayers. All monolayers were incubated at 37°C. The developing HSV-1 cytopathology was monitored daily for two days by inverted light microscopy and titers were calculated by multiple progression analysis. For the intracellular anti-viral activity, "cell monolayer controls" were obtained using sham inoculated non-treated HFF cell monolayers along with the drug treated monolayers. "Positive controls" were obtained by using HSV-1 inoculated non-CTC compound treated HFF monolayers.

### F. Results

In the tables, the symbols have the meaning indicated:
- + =: toxic;
- - =: no toxicity;
- +/- =: mild toxicity;
- ND =: not done;
- T =: toxicity effects interfered with CPE rating;
- CPE =: cytopathic effect.

### EXPERIMENT 1

### Results:

### Cell monolayer controls

No adverse HFF cellular effects were evident in these monolayers.

**TABLE 1. - TOXICITY EFFECTS CONTROLS**

| Compound | Time | Concentration (mg/ml | | | | |
|---|---|---|---|---|---|---|
| | | 10 | 1 | 0.1 | 0.01 | 0.001 |
| Non-treated | 24 | - | - | - | - | - |
| | 48 | - | - | - | - | - |
| 96-Br | 24 | Insol. | - | - | - | - |
| | 48 | Insol. | - | - | - | - |

**TABLE 2. - VIRUCIDAL EFFICACY**

| Therapy | Time | Concentration (PFU mg/ml) | | | | | |
|---|---|---|---|---|---|---|---|
| | (PI) | 0 | 10 | 1 | 0.1 | 0.01 | 0.001 |
| Untreated | 24 | 10⁶* | | | | | |
| | 48 | 10⁷ | | | | | |
| 23 | 24 | ND | 0 | 0 | 10¹ | 10² | 10³ |
| | 48 | ND | 0/T | 0 | 10⁵ | 10⁵⁻⁶ | 10⁵⁻⁶ |
| 82 | 24 | ND | 0 | 10¹⁻² | 10² | 10²⁻³ | 10⁴ |
| | 48 | ND | 0/T | 10⁵ | 10⁵⁻⁶ | 10⁵⁻⁶ | 10⁵⁻⁶ |
| 76 | 24 | ND | T | T | 10¹⁻² | 10²⁻³ | 10⁴⁻⁵ |
| | 48 | ND | T | T | 10⁵⁻⁶ | 10⁵⁻⁶ | 10⁵⁻⁶ |
| 96 | 24 | ND | Insol | 0 | 10²⁻³ | 10²⁻³ | 10²⁻³ |
| | 48 | ND | Insol | 0 | 10⁵⁻⁶ | 10⁶ | 10⁵⁻⁶ |

**TABLE 3. - EFFECTIVE VIRUCIDAL CONCENTRATION**

| Compound | IC₅₀ | IC₅₀ |
|---|---|---|
| 23 | 0.1 mg/ml | 1.0-0.1 mg/ml |
| 82 | 0.1 mg/ml | 1 mg/ml |
| 76 | 0.01 mg/ml | 0.1-1 mg/ml |
| 96 | 0.1 mg/ml | 1.0-0.1 mg/ml |

Compound 96 was insoluble at 10 mg/ml. A stock solution at a concentration of 1 mg/ml was moderately insoluble. As noted above, it was later discovered that this insolubility was due to insufficient stirring. However, a lack of toxicity of 96 was evident at 1 mg/ml and the antiviral activity was similar to that observed for 23.

### EXPERIMENT 2

### Results:

### Cell monolayer controls

No adverse HFF cellular effects were evident in these monolayers

**TABLE 4. TOXICITY EFFECTS CONTROLS**

| COMPOUND | TIME (PI) | Concentration (mg/ml) | | | |
|---|---|---|---|---|---|
| | | 1 | 0.1 | 0.01 | 0.001 |
| Untreated | 24 | - | - | - | - |
| | 48 | - | - | - | - |
| 23 | 24 | +/- | - | - | - |
| | 48 | +/- | - | - | - |
| 96 | 24 | - | - | - | - |
| | 48 | - | - | - | - |

**TABLE 5 - VIRUCIDAL EFFICACY**

| Compound | Time (PI) | Concentration (mg/ml | | | | |
|---|---|---|---|---|---|---|
| | | 0 | 1 | 0.1 | 0.01 | 0.001 |
| Untreated | 24 | 10⁶ | | | | |
| | 48 | 10⁷ | | | | |
| 23 | 24 | ND | 0 | 10¹ | 10³ | 10⁵ |
| | 48 | ND | 10⁵ | 10⁵ | 10⁵⁻⁶ | 10⁵⁻⁶ |
| 96 | 24 | ND | 0 | 10¹⁻⁶ | 10³⁻⁴ | 10⁵ |
| | 48 | ND | 10⁵ | 10⁵⁻⁶ | 10⁵⁻⁶ | 10⁶⁻⁷ |

### EXPERIMENT 3

### Cell monolayer controls

No adverse HFF cellular effects were evident in these monolayers.

Antiviral Efficacy of the compounds *in vitro.*

**TABLE 6 - VIRUCIDAL EFFICACY (24 Hours PI)**

| Compound | Concentration (mg/ml) | | | | | |
|---|---|---|---|---|---|---|
| | 5 | 1 | 05. | 01. | 0.01 | 0.00 |

| Run 1 | | | | | | |
|---|---|---|---|---|---|---|
| 93 | 0 | 0 | 10² | 10²⁻³ | 10³⁻³ | 10⁵ |
| 96 | 0 | 10¹⁻² | 10¹⁻² | 10¹⁻² | 10²⁻³ | 10⁴⁻⁵ |

| Run 2 | | | | | | |
|---|---|---|---|---|---|---|
| 93 | 0 | 0 | 0 | 0 | 10² | 10⁴⁻⁵ |
| 96 | 0 | 0 | 10¹⁻² | 10² | 10⁴ | 10⁴⁻⁵ |

| | | | | | | |
|---|---|---|---|---|---|---|
| Non-treated = 10⁵⁻⁶ | | | | | | |

**TABLE 7 - TOXICITY EFFECTS CONTROLS**

| Compound | Time hrs Post Incubation | Concentration (mg/ml) | | | | | |
|---|---|---|---|---|---|---|---|
| | | 5 | 1 | 0.5 | 0.1 | 0.01 | 0.001 |
| Untreated | no cellular effects | | | | | | |
| 93 | 24 | +/- | - | - | - | - | - |
| | 48 | +/- | - | - | +/- | - | - |
| 96 | 24 | +/- | - | - | +/- | - | - |
| | 48 | +/- | +/- | - | +/- | - | - |

**TABLE 8. EFFECTIVE VIRUCIDAL CONCENTRATION**

| Compound | IC₅₀ | IC₅₀ |
|---|---|---|
| 93 | 0.5 mg/ml | > 1.0 mg/ml |
| 96 | 0.5-0.1 mg/ml | > 1 mg/ml |

The intracellular antiviral activity of the compounds is at least one log higher than the virucidal activity. This indicates that the inventive compound has direct virucidal effect on the HSV-1 in addition to having an intracellular effect on the HSV-1. The intracellular effect appears to be non-specific and time dependent.

### EXPERIMENT 4

**TABLE 9 - VIRUCIDAL EFFICACY**

| Compound | 24 Hour Post Inoculation | | | | | |
|---|---|---|---|---|---|---|
| | 5 | 1 | 0.1 | 0.01 | 0.001 | 0.0001 |
| Run 1 | | | | | | |
| 96 | 0 | 0 | 10² | 10²⁻³ | 10³⁻⁴ | 10⁵ |
| 97 | 0 | 0 | 10¹ | 10⁴ | 10⁵ | 10⁵ |
| 102 | 0 | 0 | 10² | 10²⁻³ | 10⁴ | 10⁴⁻⁵ |
| 104 | 0 | 0 | 10² | 10³ | 10³⁻⁴ | 10⁵ |

| Run 2 | | | | | | |
|---|---|---|---|---|---|---|
| 96 | 0 | 0 | 0 | 10³ | 10³ | 103 |
| 97 | 0 | 0 | 0 | 10¹ | 10⁴ | 10⁴⁻⁵ |
| 102 | 0 | 0 | 0 | 10² | 10⁴ | 10⁴⁻⁵ |
| 104 | 0 | 0 | 0 | 10²⁻³ | 10⁴⁻⁵ | 10⁴⁻⁵ |

| | | | | | | |
|---|---|---|---|---|---|---|
| Non-treated = 10⁵ | | | | | | |

**TABLE 10 - TOXICITY EFFECTS CONTROL**

| Compound | Time hrs Post Incubation | Concentration (mg/ml) | | | | | |
|---|---|---|---|---|---|---|---|
| | | 5 | 1 | 0.5 | 0.1 | 0.01 | 0.001 |
| Untreated | no cellular effects | | | | | | |
| 96 | 24 | +/- | +/- | - | +/- | - | - |
| | 48 | + | +/- | - | +/- | - | - |
| 97 | 24 | +/- | +/- | - | +/- | - | - |
| | 48 | + | +/- | - | +/- | - | - |
| 102 | 24 | +/- | +/- | - | - | - | - |
| | 48 | + | +/- | - | - | - | - |
| 104 | 24 | +/- | +/- | - | +/- | - | - |
| | 48 | +/- | +/- | - | +/- | - | - |

**TABLE 11 - EFFECTIVE VIRUCIDAL CONCENTRATION**

| Compound | IC₅₀ | IC₉₀ |
|---|---|---|
| 96 | 0.1-1.0 mg/ml | > 1 mg/ml |
| 97 | 0.1-0.1 mg/ml | 0.1-1 mg/ml |
| 102 | 0.01-0.1 mg/ml | > 1.0 mg/ml |
| 104 | 0.1 mg/ml | > 1.0 mg/ml |

The IC₅₀ and IC₉₀ drug levels are calculated based upon the reduction in HSV titer compared to the non-drug treated control monolayers. As was observed previously, reduction in toxicity resulted in a reduction in efficacy.

### EXPERIMENT 5

### Cell monolayer controls

No adverse HFF cellular effects were evident in these monolayers.

**TABLE 12 - TOXICITY EFFECTS CONTROLS**

| Compound | 48 Hours Post Incubation Concentration (mg/ml) | | | | | | |
|---|---|---|---|---|---|---|---|
| | 5 | 2 | 1 | 0.5 | 0.1 | 0.01 | 0.001 |
| 93 | ND | ND | +/- | ++/- | ++/- | - | - |
| 96 | +/- | +/- | - | ++/- | ++/- | - | - |
| 23 | +/- | +/- | - | - | - | - | - |

**TABLE 13 - VIRUCIDAL EFFICACY (24 Hours PI)**

| Compound | Concentration (mg/ml) | | | | | | |
|---|---|---|---|---|---|---|---|
| | 5 | 2 | 1 | 0.5 | 0.1 | 0.01 | 0.001 |
| 93 | ND | ND | 0 | 0 | 10² | 10²⁻³ | 10²⁻³ |
| 96 | 0 | 0 | 0 | 0 | 10¹⁻² | 10²⁻³ | 10³⁻⁴ |
| 23 | 0 | 0 | 0 | 0 | 10⁰⁻¹ | 10¹⁻² | 10³⁻⁴ |

| | | | | | | | |
|---|---|---|---|---|---|---|---|
| Non-treated = 10⁴ | | | | | | | |

**TABLE 14 - EFFECTIVE VIRUCIDAL CONCENTRATION**

| Compound | IC₅₀ |
|---|---|
| 93 | 0.1 mg/ml |
| 96 | 0.1 mg/ml |
| 23 | 0.01 mg/ml |

The IC₅₀ drug level was calculated based upon the reduction in HSV titer compared to the non-drug treated control monolayers. Reduction in toxicity (e.g. 93 and 96) resulted in a reduction in efficacyof the compounds in this virucidal assay. The toxicity observed with 93 and 96 was demonstrated again. The toxicity appears to be more pronounced in the 0.5 and the 0.1 mg/ml concentrations. The toxicity was evident as cell rounding, detaching from the monolayer, increased granularity and loss of normal morphology. At concentrations below the 0.5 and 0.1 mg/ml, the cell monolayers appeared (visually) to be normal with no overt toxic effects.

### EXPERIMENT 6

**TABLE 15 - ANTIVIRAL EFFICACY**

| Compound | 24 Hour Post Inoculation Efficacy PFU/ml Concentration (mg/ml | | | | | | |
|---|---|---|---|---|---|---|---|
| | 5 | 2 | 1 | 0.5 | 0.1 | 0.01 | 0.001 |
| 93 | ND | ND | 0 | 10² | 10²⁻³ | 10³⁻⁴ | 10⁵ |
| 96 | 0 | 0 | 10¹⁻² | 10¹⁻² | 10² | 10²⁻³ | 10⁴⁻⁵ |
| 23 | 0 | 0 | 0 | 0-10¹ | 10¹⁻² | 10² | 10³⁻⁴ |

| | | | | | | | |
|---|---|---|---|---|---|---|---|
| Non-treated = 10⁵ | | | | | | | |

**TABLE 16 - EFFECTIVE VIRUCIDAL CONCENTRATION**

| Compound | IC₅₀ |
|---|---|
| 93 | 0.5 mg/ml |
| 96 | 0.5-10.0 mg/ml |
| 23 | 0.1 mg/ml |

Intracellular antiviral activity of the compounds is a minimum of one log higher than the virucidal activity.

### EXAMPLE 2

### A. VIRUS USED

VZV was grown to a CPE level *in vitro* assays with VZV of 3-4 + on HFF cell monolayers. The VZV was scraped from the flask, centrifuged and the cell pellet was resuspended in 10 ml of complete medium. 100 to 200 µl of the VZV cell associated inoculum was inoculated onto confluent HFF cell monolayers by absorption for 60 minutes at 37°C. After absorption of the virus, the inoculum was aspirated from the cell monolayers.

### B. PREPARATION OF SOLUTIONS TO BE TESTED

Concentrations of compounds 93, 96, and 23 were prepared as follows: A stock solution of each compound at a concentration of 5 mg/ml was made. This stock solution was serially diluted in Hank's Balanced Salt Solution to obtain final drug concentrations of: 5, 2, 1, 0.5, 0.1, 0.01, 0.001, and 0.0001 mg/ml . 500 µl of each drug concentration were added to triplicate infected monolayers. (Compound 93 was observed not to be soluble at 5 and 2 mg/ml concentrations. The highest concentration of this compound used in these assays was 1 mg/ml. As explained earlier, this was due to insufficient stirring.)

### C. INOCULATION AND ANALYSIS

The VZV-infected monolayers were rehydrated with medium containing the appropriate concentrations of compounds to be tested. All monolayers were incubated at 37°C. Development of VZV cytopathology was monitored daily for 7 days by inverted light microscopy. Titers were calculated by multiple regression analysis.

### CONTROLS

Sham-inoculated, non-treated HFF cell monolayers were included among with the drug-treated monolayers as cell monolayer controls.

VZV inoculated, non-treated HFF monolayers were included as positive controls.

### RESULTS

### Cell monolayer controls

No adverse HFF cellular effects were evident in these monolayers.

**TABLE 17 - VIRUCIDAL EFFICACY**

| Compound | 7 Days Post Inoculation Efficacy: PFU/ml Concentration (mg/ml | | | | | | |
|---|---|---|---|---|---|---|---|
| | 5 | 2 | 1 | 0.5 | 0.1* | 0.01 | 0.001 |
| 93 | ND | ND | T | T | T? | 10² | 10⁴ |
| 96 | 0 | 0 | T | T | T? | 10² | 10⁴ |
| 23 | 0 | 0 | 0 | 0 | T? | 10¹⁻² | 10²⁻³ |

| | | | | | | | |
|---|---|---|---|---|---|---|---|
| Non-treated = 10⁴ *) This toxicity observation is questionable. | | | | | | | |

**TABLE 18 - EFFECTIVE VIRUCIDAL CONCENTRATION**

| Compound | IC₅₀ |
|---|---|
| 93 | 0.01 mg/ml |
| 96 | 0.01 mg/ml |
| 23 | 0.01-0.001 mg/ml |

Figure 1 shows the effects of Compound 96 on survival in a mouse genital HSV-2 model. Mice received one treatment of intravaginal saline (placebo), 2 % Acyclovir, 0.5% Compound 96, or 2 % Compound 96 as shown, just prior to infection.

### EXAMPLE 4

A series of viral screening tests were carried out for Compounds 23, 64, 67, 93, 96, and 102. The structure of compounds number 64 and 67 are set forth in U.S. Patent No. 5,049,557 at paragraphs 2 and 3 of the Experiment Details section. In particular, for Compound 64, A is phenyl, Y is hydrogen, B is methyl, Z-is chloride, and X and X' are NH₃. For Compound 67, A is methyl, B is methyl, Y is chlorine, Z- is chloride, and X and X' are NH₃. Compounds 23, 64, 67, 93 and 102 were screened for comparison purposes. The results are shown in Table 19.

The following procedures were utilized for determining antiviral efficacy and toxicity.

### A. Preparation of Human Foreskin Fibroblast Cells

Newborn human foreskin were obtained as soon as possible after circumcisions were performed and placed in minimal essential medium (MEM) containing vacomycin, fungizone, penicillin, and gentamycin, at the usual concentrations, for four hours. The medium was then removed, the foreskin minced into small pieces and washed repeatedly until red cells were no longer present. The tissue was then trypsinized using trypsin at 0.25% with continuous stirring for 15 minutes at 37°C in a CO₂ incubator. At the end of each 15 minute period, the tissue was allowed to settle to the bottom of the flask. The supernatant containing cells was poured through sterile cheesecloth into a flask containing MEM and 10% fetal bovine serum. The flask containing the medium was kept on ice through out the trypsinizing procedure. After each addition of cells, the cheesecloth was washed with a small amount of MEM containing serum. Fresh trypsin was added each time to the foreskin pieces and the procedure repeated until no more cells became available. The cell containing medium was then centrifuged at 1000 RPM at 4°C for ten minutes. The supernatant liquid was discarded and the cells resuspended in a small amount of MEM with 10% FBS. The cells were then placed in an appropriate number of 25 cm² tissue culture flasks. As cells became confluent and needed trypsinization, they were gradually expanded into larger flasks. The cells were kept on vancomycin and fungizone to passage four.

### B. Cytopathic Effect Inhibition Assay

Low passage human foreskin fibroblast cells were seeded into 96 well tissue culture plates 24 hours prior to use at a cell concentration of 2.5 x 10⁴ cells per ml in 0.1 ml (MEM) supplemented with 10% fetal bovine serum (FBS). The cells were then incubated for 24 hours at 37°C in a CO₂ incubator. After incubation, the medium was removed and 100 µl of MEM containing 2 % FBS was added to all but the first row. In the first row, 125 µl of experimental drug was added in triplicate wells. Medium alone was added to both cell and virus control wells. The drug in the first row of wells was then diluted serially 1:5 throughout the remaining wells by transferring 25 µl using the Cetus Liquid Handling Machine. Afer dilution of drug, 100 µl of the appropriate virus concentration was added to each well, excluding cell control wells which received 100 µl of MEM. For HSV-1 and HSV-2 assays, the virus concentration utilized was 1000 PFU's per well. For CMV and VZV assays, the virus concentration added was 2500 PFU per well. The plates were then incubated at 37°C in a CO₂ incubator for three days for HSV-1 and HSV-2, 10 days for VZV, or 14 days for CMV. After the incubation period, media was aspirated and the cells stained with a 0.1 % crystal violet solution for 30 minutes. The stain was then removed and the plates rinsed using tap water until all excess stain was removed. The plates were allowed to dry for 24 hr and then read on a Skatron Plate Reader at 620 nm.

### C. Plaque Reduction Assay for HSV-1 and HSV-2 Using Semi-Solid Overlay

Two days prior to use, HFF cells are plated into six well plates and incubated at 37°C with 5% CO₂ and 90% humidity. On the date of assay, the drug is made up at twice the desired concentration in 2X MEM and then serially diluted 1:5 in 2X MEM using six concentrations of drug. The initial starting concentration isusually 200 µg/ml down to 0.06 µg/ml. The virus to be used is diluted in MEM containing 10% FBS to a desired concentration which will give 20 - 30 plaques per well. The media is then aspirated from the wells and 0.2 ml of virus is added to drug toxicity wells. The plates are then incubated for one hour with shaking every fifteen minutes. After the incubation period, an equal amount of 1% agarose was added to an equal volume of each drug dilution. This will give final drug concentrations beginning with 100 µg/ml and ending with 0.03 µg/ml and a final agarose overlay concentration of 0.5%. The drug agarose mixture is applied to each well in 2 ml volume and the plates then incubated for three days, after which the cells were stained with a 1.5 % solution of neutral red. At the end of 4-6 hr incubation period, the stain is aspirated, and plaques counted using a stereomicroscope at 10X magnification.

### D. VZV Plaque Reduction Assay - Semi-Solid Overly

The procedure is essentially the same as for the HSV plaque assay described above with two exceptions:
1. After addition of the drug, the plates are incubated for ten days.
2. On days three and six an additional 1 ml overlay with equal amounts of 2X MEM and 1% agarose are added.

### E. CMV Plaque Assay - Semi-Solid Overlay

The procedure is essentially the same as for HSV analysis with the following minor changes. The agarose used for the initial overlay and the two subsequent overlays is 0.8 % rather than 1%. They assay is incubated for 14 days with the additional 1 ml overlays being applied on days four and eight.

### F. Plaque Reduction Assays Using Liquid Medium Overlay

The procedure for the liquid overlay plaque assay is similar to that using the agarose overlay. The procedure for adding the virus is the same as for the regular plaque assay. The drugs are made up in a concentration to be used in MEM with 2% FBS. The drugs are not made up at 2X concentration as in previous assays but are made up at the desired concentration. For HSV-1 and HSV-2 assays, an antibody preparation obtained from Baxter Health Care Corporation is diluted 1:500 and added to the media that the drug is diluted in. For CMV and VZV, no antibody in the overlay is utilized. For the CMV assay, additional medium without new drug is added on day six and allowed to incubate for a total of 11 days. For VZW, additional media is added on day five and incubated for a total of eight days. At the end of the incubation period for all of the assays, the medium is removed, the cells washed and then stained with 0.1% crystal violet solution for ten minutes. The cells are then rinsed several times to remove any excess violet and plaques enumerated using a stereomicroscope.

### G. Cell Proliferation Assay

Twenty-four hours prior to assay, HFF cells are seeded in 6-well plates at a concentration of 2.5 x 10⁴ cells per well in MEM containing 10% FBS. On the day of the assay, drugs are diluted serially in MEM containing 10% FBS at increments of 1:5 covering a range from 100 µg/ml to 0.03 µg/ml. For drugs that have to be solubilized in DMSO, control wells receie MEM containing 10% DMSO. The media from the wells is then aspirated and 2 ml of each drug concentration is then added to each well. The cells are then incubated in a CO₂ incubator at 37°C for 72 hours. At the end of this time, the media-drug solution is removed and the cells washed. One ml of 0.25% trypsin is added to each well and incubated until the cells start to come off of the plate. The cell-media mixture is then pipetted up and down vigorously to break up the cell suspension and 0.2 ml of the mixture is added to 9.8 ml of Isoton III and counted using a Coulter Counter. Each sample is counted three times with three replicate wells per sample.

### H. MTT Assay for Cell Cytotoxicity

Twenty-four hours prior to assay, HFF cells are plated into 96 well plates at a concentration of 2.5 x 10⁴ cells per well. After 24 hours, the media is aspirated and 1256 microliters of drug is added to the first row of wells and then diluted serially 1:5 using the automated Cetus Liquid Handling System in a manner similar to that used in the CPE assay. The plates are then incubated in a CO₂ incubator at 37°C for seven days. At this time, each well receives 50 microliters of 1 µg/ml solution of MTT in Dulbecco's Phosphate Buffered Saline. The plates are then incubated for an additional four hours. At this time, the media is removed and replaced with 100 µl of 0.04 N hydrochloric acid in isopropanol. After shaking briefly, the plates are then read on a plate reader at 550 nm.
1. EC50 values were also determined for each of the viruses tested for known antiviral agents. The comparison compound was ACV for all viruses except for HCMV wherein DHPG was the comparison compound. In Table 19, these comparison EC50 values are indicated with an asterisk. In Table 20, the drug FIAU (2'-fluoro-5-iodo-arabinosyl-uracil) was used as a control.

**TABLE 19**

| ANTIVIRAL ACTIVITY | | | | | | | | | | |
|---|---|---|---|---|---|---|---|---|---|---|
| Compound | VIRUS | | | | | | | | | |
| | | HSV-1 | | HSV-2 | | HCMV | | VZV | | EBV |
| | | CPE | Plaque | CPE | Plaque | CPE | Plaque | CPE | Plaque | IMN |
| 23 | EC50 | 16.5 | >4 | 4.6 | >4 | 14.6 | 15.6 | | >4 | 2.7 |
| | IC50 | >86 | 7.0 | >86 | 7.0 | >97 | 26.2 | | 11 | 1.9 |
| | SI | >5.2 | <1.7 | >19 | <1.7 | >6.6 | 1.7 | | <2.7 | 0 |
| | EC50 | .20 | .2 | .70 | .0 | .1 .9 | | | 3.2 | 5.8 |
| 64 | EC50 | 10.3 | | 6.2 | | 52.4 | >4 | | >4 | 2.7 |
| | IC50 | >87 | | >87 | | >73 | 9.9 | | 18 | 4.5 |
| | SI | >8.4 | | >14 | | >1.4 | 2.5 | | >4.5 | 1.7 |
| | EC50 | .20 | | .70 | | .10 | 0.7 | | 3.2 | 6.0 |
| 67 | EC50 | 3.2 | | 1.7 | | >100 | | | >2 | 0.6 |
| | IC50 | >100 | | >100 | | >79 | | | 11.5 | 3.6 |
| | SI | >31 | | >60 | | 0 | | | <5.7 | 6.0 |
| | EC50 | .20 | | .70 | | 0.8 | | | 4.4 | 3.6 |
| 93 | EC50 | 29.0 | | 6.1 | | >52 | 44.7 | | >3.3 | 2.7 |
| | IC50 | >94 | | >94 | | >100 | 41.4 | | 8.2 | 2.4 |
| | SI | >3.2 | | >15 | | <1.9 | 0 | | <2.5 | 0 |
| | EC50 | .20 | | .70 | | 0.8 | 0.73 | | 3.1 | 3.6 |
| 96 | EC50 | 6.8 | >4 | 5.3 | >4 | 17.5 | | | >4 | 1.3 |
| | IC50 | 80.0 | 10.2 | 80.0 | 10.2 | >83 | | | 26 | 7.3 |
| | SI | 12 | <2.5 | 15 | <2.5 | >4.7 | | | <6.5 | 5.6 |
| | EC50 | 0.20 | 0.2 | 0.70 | 0.9 | 0.8 | | | 3.0 | 3.6 |
| 102 | EC50 | 1.7 | 5.6 | 5.0 | 2.2 | 33 | | | >12 | 0.67 |
| | IC50 | >92 | 11.5 | >92 | 11.5 | >100 | | | 27 | 2.2 |
| | SI | >54 | 2.0 | >18 | 5.2 | >3.3 | | | <2.2 | 3.3 |
| | EC50 | 0.20 | 0.2 | 0.70 | 0.3 | 0.05 | | | 3.0 | 3.6 |

**TABLE 20**

| TOXICITY ASSAYS - IC₅₀ | | | |
|---|---|---|---|
| Compound | NEUTRAL RED UPTAKE (MCG/ML) (STATIONARY CELLS) | MTT TOXICITY (MCG/ML) (STATIONARY CELLS) | CELL PROLIFERATION (MCG/ML) (RAPIDLY GROWING CELLS) |
| 23 | 11.5 | >61 | 6.2 |
| FIAU | - | - | 13.1 |
| 64 | 15.2 | 53 | 38 |
| FIAU | - | - | 8.4 |
| 67 | 12.1 | 42.0 | 12.0 |
| FIAU | - | - | 8.4 |
| 93 | 27.3 | 65.0 | 25.0 |
| FIAU | - | - | 8.4 |
| 96 | 22.0 | > 74 | 21.0 |
| FIAU | - | - | 3.3 |
| 102 | 23.2 | >68 | 15 |
| FIAU | - | - | 3.3 |

### EXAMPLE 5

The compound may be prepared by the following general procedure. The cobalt-II complex is prepared by mixing equimolar amounts of the N,N'-bisethylenediimine ligands, e.g., L23 and the like as disclosed in U.S. Patent No. 5,049,557 with cobalt acetate in methanol under nitrogen. About 2.2 equivalents of the desired axial ligand is added followed by oxidation. The desired product may then be precipitated by the addition of a saturated aqueous solution of sodium chloride or sodium bromide followed by recrystallization from an ethanol-water solution.

Compound 96 (having bromide as the counterion) was synthesized as follows:
A 3-neck flask equipped with a nitrogen bubbler and a 2 liter dropping funnel was charged with 112 grams (0.5 moles) of the ligand (L23 or N,N'bis-(acetylacetone)ethylene-diimine) in 500 ml of absolute methanol. To the ligand solution is added 125 grams (0.5 moles) of cobalt acetate tetrahydrate dissolved in 1.5 litersof degassed methanol. The reaction mixture is stirred for 2 hours and then refluxed for 15 minutes on a hot water bath. An orange solution results to which 90 grams (1.1 moles) of 2-methyl imidazole dissolved in 100ml of methanol are added. The reaction mixture is exposed to the open air while maintaining vigorous stirring. Ten grams of activated charcoal are added to the stirring mixture and the oxidation is continued overnight.

The mixture is then filtered and 50 grams of sodium bromide dissolved in a minimum amount of water is added to the filtered brown solution. The solution obtained is concentrated and allowed to crystallize. The crude product is recrystallized from hot ethanol-water solution by standing at room temperature or a lower temperature. The purity of the product is checked by elemental analysis, electronic spectra and NMR.

### EXAMPLE 6

In a study of the mouse model, vaginal herpes infection was completely blocked by topical application of Compound 96. Sixty female Swiss Webster mice pretreated with medroxyprogesterone acetate were randomized to four groups of 15 to receive either Saline (control), 2 % Acyclovir, 2 % Compound 96, or 0.5% Compound 96. Animals were anesthetized by intraperitoneal injection of sodium pentabarbital and then administered 15 µl of control or test compound intravaginally in one treatment. They were then immediately challenged by intravaginal instillation with 15 µl of a suspension containing 10⁴ plaque forming units HSV-2 strain 186. Vaginal swabs were collected on days 1 and 2 post-inoculation (PI) to assess the effect of treatment on vaginal replication in the genital tract. In addition, all animals were examined daily until day 21 PI for mortality and signs of infection (erythema, hair loss and hind limb paralysis).

The mortality curve of the experiment is shown in Figure 1, and final outcome data in Table 21. Both the control and acyclovir groups showed high mortality, with 13/15 and 12/15 mice dying, respectively. In contrast, the Compound 96 groups all lived, and none of these animals developed any signs of genital disease.

Viral replication data are also presented in Table 21. Virus was found in the genital tracts of all 15 control-treated mice, indicating that all the animals, including the two survivors, became infected. Among the mice receiving acyclovir, virus was detected in 12/15 animals, indicating that intravaginal acyclovir did not provide significant protection against infection. Mice treated with Compound 96 had no detectable virus on either day 1 or 2 PI, showing that treatment prevented virus replication at the site of inoculation.

**TABLE 21**

| Treatment | Number | Dead | Virus Isolated | Vaginal Virus Titer Day 1^{a} | Vaginal Virus Titer Day 2^{a} |
|---|---|---|---|---|---|
| Saline | 15 | 13 | 15 | 2.21 ± 0.27 | 3.64 ± 0.24 |
| 2% Acyclovir | 15 | 12 | 12 | 1.96 ± 0.38 | 3.82 ± 0.38 |
| 2% Cmpd 96 | 15 | 0 | 0 | 0 | 0 |
| 0.5% Cmpd 96 | 15 | 0 | 0 | 0 | 0 |

| | | | | | |
|---|---|---|---|---|---|
| ^{a} mean log₁₀ viral titers ± SEM are averaged for infected animals only | | | | | |

The study was repeated using a wider range of Compound 96 concentrations. The lowest concentration of 0.01 % protected 6 of 10 animals from death, while concentrations of 0.1 % or more completely protected mice from visible infection and death. The results of this study are presented in Table 22.

**TABLE 22**

| Treatment | Number | Dead | Virus Isolated | Vaginal Virus Titer Day 2^{a} |
|---|---|---|---|---|
| Saline | 13 | 13 | 13 | 3.8 ± 0.2 |
| 2% Cmpd 96 | 10 | 0 | 0 | 0 |
| 0.5% Cmpd 96 | 10 | 0 | 0 | 0 |
| 0.1 % Cmpd 96 | 10 | 0 | 0 | 0 |
| 0.03% Cmpd 96 | 10 | 2 | 1 | 5.52 ± 0 |
| 0.01 % Cmpd 96 | 10 | 4 | 5 | 3.9 ± 0.8 |

| | | | | |
|---|---|---|---|---|
| ^{a} mean log₁₀ viral titers ± SEM are averaged for infected animals only | | | | |

A study of the effect of the time of application relative to the time of inoculation with the virus is reported in Table 23.

**TABLE 23**

| Time of application | Number | Symptomatic | Dead |
|---|---|---|---|
| 0 time | 10 | 0 | 0 |
| 5 minutes pre | 10 | 0 | 0 |
| 1 hour pre | 10 | 4 | 3 |
| 3 hours pre | 10 | 7 | 7 |
| 6 hours post | 10 | 3 | 3 |
| Saline | 10 | 7 | 7 |

## Claims

1. Use of a compound in the manufacture of a composition for use in the prevention of herpes virus infection in animals by topical application, said compound having the structure. 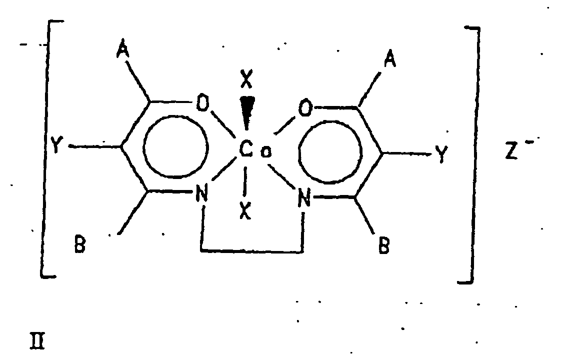 wherein each
A is a methyl group;
Y is hydrogen;
B is a methyl group
Z is a bromine ion, and
X is a moiety having the formula: 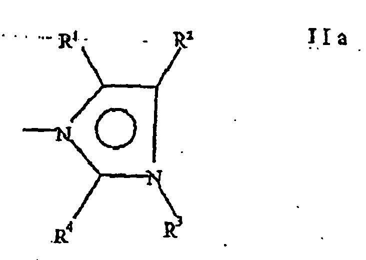
wherein each of R¹, R² and R³ is hydrogen and R⁴ is a methyl group;

2. Use according to Claim 1 wherein the composition is in the form of a pharmaceutically acceptable saline solution, ointment, salve, creme.

3. Use according to Claim 1 or 2 wherein the composition is applied to that site on the animal which is exposed to the herpes virus.

4. Use according 10 Claim 3 wherein the composition is applied intravaginally.

5. Use according to Claim 3 or 4 wherein the composition is applied from 1 hour before to 6 hours after exposure to the herpes virus.

6. Use according to Claim 5 wherein the composition is applied from 5 minutes before to 5 minutes after exposure to the herpes virus.

7. Use according to any one of the preceding claims wherein the herpes virus is Herpes Simplex Virus-2.

8. Use according to any one of the preceding claims wherein topical application of the composition is performed by contacting the animal with an applicator coated with the composition.

9. Use according to Claim 8 wherein the applicator is a condom.

10. A compound for use in the prevention of herpes virus infection in animals by topical application, said compound having the structure 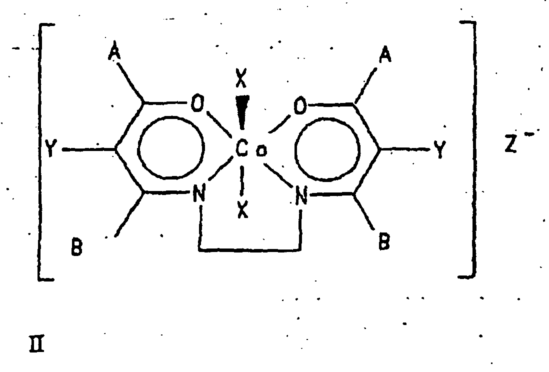 wherein each
A is a methyl group;
Y is hydrogen;
B is a methyl group
Z is a bromine ion, and
X is a moiety having the formula: 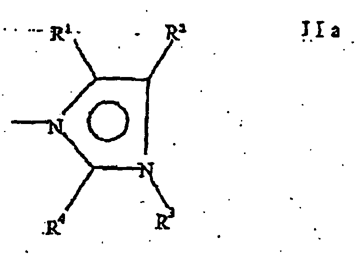
wherein each of R¹, R² and R³ is hydrogen and R⁴ is a methyl group;

11. A compound for use according to claim 10 wherein the compound is formulated as a composition in the form of a pharmaceutically acceptable saline solution, ointment, salve or cream.

## Patentansprüche

1. Verwendung einer Verbindung bei der Herstellung einer Zusammensetzung zur Verwendung bei der Prävention einer Herpesvirus-Infektion bei tierischen Lebewesen durch topische Applikation, wobei die Verbindung die folgende Struktur aufweist: 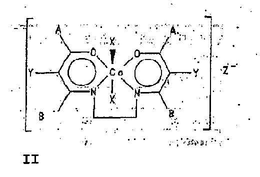 wobei jedes
A für eine Methylgruppe steht,
Y für Wasserstoff steht,
B für eine Methylgruppe steht,
Z für einen Bromion steht und
X für eine Einheit der folgenden Formel steht: 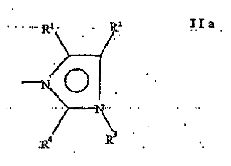
wobei jeder Rest R¹, R² und R³ für Wasserstoff steht und R⁴ für eine Methylgruppe steht.

2. Verwendung nach Anspruch 1, wobei die Zusammensetzung in der Form einer pharmazeutisch akzeptablen Kochsalzlösung, eines pharmazeutisch akeptablen Unguentums, einer pharmazeutisch akzeptablen Salbe oder einer pharmazeutisch akzeptablen Creme vorliegt.

3. Verwendung nach Anspruch 1 oder 2, wobei die Zusammensetzung an der Stelle an dem tierischen Lebewesen appliziert wird, an der die Exposition gegenüber dem Herpesvirus erfolgt.

4. Verwendung nach Anspruch 3, wobei die Zusammensetzung intravaginal appliziert wird.

5. Verwendung nach Anspruch 3 oder 4, wobei die Zusammensetzung 1 Stunde vor bis 6 Stunden nach der Exposition gegenüber dem Herpesvirus appliziert wird.

6. Verwendung nach Anspruch 5, wobei die Zusammensetzung 5 Minuten vor bis 5 Minuten nach der Exposition gegenüber dem Herpesvirus appliziert wird.

7. Verwendung nach einem der vorhergehenden Ansprüche, wobei das Herpesvirus das Herpes simplex-Virus 2 ist.

8. Verwendung nach einem der vorhergehenden Ansprüche, wobei eine topische Applikation der Zusammensetzung durch Inkontaktbringen des tierischen Lebewesens mit einem mit der Zusammensetzung beschichteten Applikator durchgeführt wird.

9. Verwendung nach Anspruch 8, wobei der Applikator ein Kondom ist.

10. Verbindung zur Verwendung bei der Prävention einer Herpesvirus-Infektion bei tierischen Lebewesen durch topische Applikation, wobei die Verbindung die folgende Struktur aufweist: 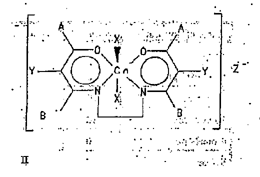 wobei jedes
A für eine Methylgruppe steht,
Y für Wasserstoff steht,
B für eine Methylgruppe steht,
Z für einen Bromion steht und
X für eine Einheit der folgenden Formel steht: 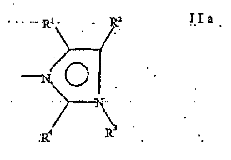
wobei jeder Rest R¹, R² und R³ für Wasserstoff steht und R⁴ für eine Methylgruppe steht.

11. Verbindung zur Verwendung nach Anspruch 10, wobei die Verbindung als eine Zusammensetzung in der Form einer pharmazeutisch akzeptablen Kochsalzlösung, eines pharmazeutisch akeptablen Unguentums, einer pharmazeutisch akzeptablen Salbe oder einer pharmazeutisch akzeptablen Creme formuliert ist.

## Revendications

1. Utilisation d'un composé dans la fabrication d'une composition pour utilisation dans la prévention d'une infection par l'herpèsvirus chez des animaux par application topique, ledit composé ayant la structure 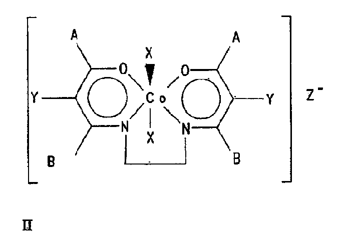 dans laquelle chaque A est un groupe méthyle ;
Y est un hydrogène ;
B est un groupe méthyle Z est un ion de brome, et
X est un fragment ayant la formule : 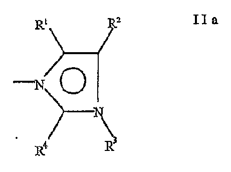 dans laquelle chacun de R¹, R² et R³ est un hydrogène et R⁴ est un groupe méthyle.

2. Utilisation selon la revendication 1, **caractérisée en ce que** la composition est sous la forme d'une solution saline, d'une pommade, d'une salve, d'une crème pharmaceutiquement acceptable.

3. Utilisation selon la revendication 1 ou 2, **caractérisée en ce que** la composition est appliquée au site sur l'animal qui est exposé à l'herpèsvirus.

4. Utilisation selon la revendication 3, **caractérisée en ce que** la composition est appliquée par voie intravaginale.

5. Utilisation selon la revendication 3 ou 4, **caractérisée en ce que** la composition est appliquée de 1 heure avant à 6 heures après l'exposition à l'herpèsvirus.

6. Utilisation selon la revendication 5, **caractérisée en ce que** la composition est appliquée de 5 minutes avant à 5 minutes après l'exposition à l'herpèsvirus.

7. Utilisation selon l'une quelconque des revendications précédentes, **caractérisée en ce que** l'herpèsvirus est le virus herpès simplex 2.

8. Utilisation selon l'une quelconque des revendications précédentes, **caractérisée en ce que** l'application topique de la composition est effectuée par mise en contact de l'animal avec un applicateur enrobé avec la composition.

9. Utilisation selon la revendication 8, **caractérisée en ce que** l'applicateur est un préservatif.

10. Composé pour utilisation dans la prévention d'une infection par l'herpèsvirus chez des animaux par application topique, ledit composé ayant la structure 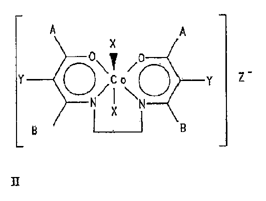 dans laquelle chaque
A est un groupe méthyle ;
Y est un hydrogène ;
B est un groupe méthyle Z est un ion brome, et
X est un groupe caractéristique ayant la formule : 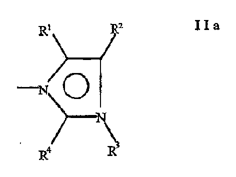 dans laquelle chacun de R¹, R² et R³ est un hydrogène et R⁴ est un groupe méthyle.

11. Composé pour utilisation selon la revendication 10, **caractérisé en ce que** le composé est formulé en tant que composition sous la forme d'une solution saline, d'une pommade, d'une salve ou d'une crème pharmaceutiquement acceptable.
